# EUROPEAN PATENT APPLICATION

(11) **EP 1 892 234 A1**
(43) Date of publication of application: **27.02.2008**
(21) Application number: 06766522.4
(22) Date of filing: 09.06.2006
(51) Int. Cl.: C07C 211/56, C07F 5/02

(54) **NOVEL ARYL COMPOUND**

(30) Priority: 10.06.2005 JP 2005171069
(71) Applicant: Sumitomo Chemical Company, Limited, Tokyo 104-8260 (JP)
(72) Inventor: OHUCHI, Kazuei, 3050005 (JP); HIGASHIMURA, Hideyuki, 3050045 (JP); FUKUSHIMA, Daisuke, 3050045 (JP)
(74) Representative: Vossius & Partner
(86) International application number: PCT/JP2006/311610
(87) International publication number: WO 2006/132357

(57) **Abstract**

A novel aryl compound represented by the formula (1) in which the characteristic groups represented by X¹ and X² are different from each other: 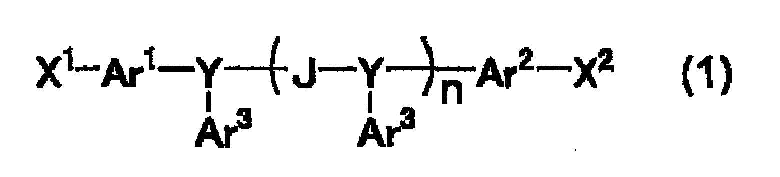 wherein X¹ and X² are characteristic groups which are different from each other and each of which is selected from the group consisting of a chlorine, bromine or iodine atom, -OSO₂Q¹, -B(OQ²)₂, -Si(OQ³)₃, -Sn(Q⁴)₃, Z¹(Z²)m, a metal acetylide group and a terminal acetylene group (where Q represents a hydrocarbon group; Q² represetns a hydrogen atom or a hydrocarbon gruop; two Q²'s may be the same as or different from each other or may together form a ring; Q³ represents a hydrocarbon group and three Q³'s may be the same as or different from one another; Q⁴ represetns a hydrocarbon group and three Q⁴'s may be the same as different from one another; Z¹ represents a metal atom or a metal ion; Z² represents a counter anion; and m is an integer of 0 or higher); Ar¹ and Ar² independently represent an arylene group and Ar³ represetns an aryl group, provided that any two of Ar¹, Ar² and Ar³ may be bound to each other via a substituent or directly; Y represents a trivalent atomic group having a nitrogen, carbon, boron or silicon atom to which a hydrogen atom or a hydrocarbon group may be added if required; J represents an arylene group; and n is an integer of 0 or higher; provided that, when each of J, Y and Ar³ is present in the molecule in plural number, they may be the same as or different from each other or one another.

## Description

### TECHNICAL FIELD

The present invention relates to a novel aryl compound having two characteristic groups different from each other, and its manufacturing method.

### BACKGROUND ART

Aryl compounds are known to be useful as raw materials for functional materials, such as organic EL materials and conductive polymer materials, ligands in catalytic reaction and the like due to their electronic characteristics and steric characteristics (see Patent Documents 1 to 3).

Recently, in the development of polymeric luminescent materials and the like, incorporation of an aryl compound into a polymer compound has been carried out by introducing a characteristic group into the aryl compound and making it react (see Non-Patent Document 1).

Patent Document 1: JP-A-2004-292782 (US 2004-262574A1)
Patent Document 2: JP-A-2003-147347 (US 2003-51633A1)
Patent Document 3: JP-A-2003-155476 (US 2003-64247A1)
Non-Patent Document 1: Organic Electronics 2003, 4, 49-59

### DISCLOSURE OF THE INVENTION

### Problem to be Solved by the Invention

However, the above-mentioned aryl compounds have had a problem of constituting the terminal structure or side chain structure of the resulting polymer compounds since they have only one characteristic group introduced. Or, the aryl compound may have two or more characteristic groups introduced and may be able to be introduced as a main chain structure into a polymer compound. Even so, the characteristic groups are the same, and thus a significant difference in reactivity between them cannot be provided. Consequently, such aryl compounds have had a problem that advanced structural features such as their direction, positions, unit number introduced and sequence length in the resulting polymer compound are difficult to sufficiently control.

It is an object of the present invention to provide a novel aryl compound having two characteristic groups different from each other which is useful as raw material for functional materials such as organic EL materials and conductive polymer materials, whose structures are highly controlled, polymer-supported catalysts or the like.

The compound of the present can be easily derivatized into a polymeric or low molecular compound with a highly controlled structure by utilizing a difference in reactivity between the two different characteristic groups in various chemical reactions.

### Means for Solving the Problem

As a result of exhaustive studies to solve the above-mentioned problems, the present inventors have found a novel aryl compound having two characteristic groups different from each other, and have completed the present invention.

Specifically, the present invention provides a novel aryl compound represented by the following general formula (1) and having mutually different characteristic groups denoted by X¹ and X².

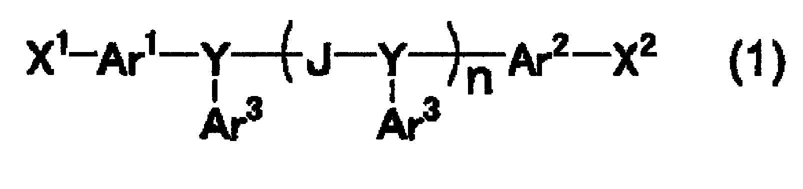

(wherein, X¹ and X² each independently denotes one characteristic group selected from the group consisting of a chlorine atom, a bromine atom, an iodine atom, -OSO₂Q¹, -B(OQ²)₂, -Si(OQ³)₃, -Sn(Q⁴)₃, Z¹(Z²)m, a metal acetylide group and a terminal acetylene group; (herein, Q¹ is a hydrocarbon group which may have a substituent; Q² is a hydrogen atom or a hydrocarbon group which may have a substituent, two Q²s may be the same or different, and may bond with each other to form a ring; Q³ is a hydrocarbon group which may have a substituent, and three Q³s may be the same or different; Q⁴ is a hydrocarbon group which may have a substituent, three Q⁴s may be the same or different; Z¹ is a metal atom or a metal ion, Z² is a counter anion thereof; and m denotes an integer of not less than 0); Ar¹ and Ar² each independently denotes an arylene group, and Ar³ denotes an aryl group; Ar¹, Ar² and Ar³ may be bonded with one another directly or through a substituent; Y denotes a trivalent atom group in which a hydrogen atom or a hydrocarbon group is optionally added to a nitrogen atom, carbon atom, boron atom or silicon atom; J denotes an arylene group; n denotes an integer of not less than 0; and in the case where J, Y and Ar³ are each present in plural numbers, they may be each the same or different from one another.)

The novel aryl compound having two mutually different characteristic groups according to the present invention is useful as functional materials such as organic EL materials and conductive polymer materials, whose structures are highly controlled, and raw materials of catalytic reaction ligands and the like.

### BEST MODE FOR CARRYING OUT THE INVENTION

The novel aryl compound having two mutually different characteristic groups according to the present invention has a structure represented by the above general formula (1).

Hydrocarbon groups in the groups denoted by Q¹, Q², Q³ and Q⁴ in the characteristic groups denoted by X¹ and X² in the above-mentioned general formula include, for example, alkyl groups having about 1 to 50 carbon atoms such as a methyl group, ethyl group, propyl group, isopropyl group, butyl group, isobutyl group, t-butyl group, pentyl group, hexyl group, a nonyl group, dodecyl group, pentadecyl group, octadecyl group and a docosyl group; cyclic saturated hydrocarbon groups having about 3 to 50 carbon atoms such as a cyclopropyl group, cyclobutyl group, cyclopentyl group, cyclohexyl group, cyclononyl group, cyclododecyl group, norbornyl group and adamantyl group; alkenyl groups having about 2 to 50 carbon atoms such as an ethenyl group, propenyl group, 3-butenyl group, 2-butenyl group, 2-pentenyl group, 2-hexenyl group, 2-nonenyl group and 2-dodecenyl group; aryl groups having about 6 to 50 carbon atoms such as a phenyl group, 1-naphthyl group, 2-naphthyl group, 2-methylphenyl group, 3-methylphenyl group, 4-methylphenyl group, 4-ethylphenyl group, 4-propylphenyl group, 4-isopropylphenyl group, 4-butylphenyl group, 4-t-butylphenyl group, 4-hexylphenyl group, 4-cyclohexylphenyl group, 4-adamantylphenyl group and 4-phenylphenyl group; and aralkyl groups having about 7 to 50 carbon atoms such as a phenylmethyl group, 1-phenyleneethyl group, 2-phenylethyl group, 1-phenyl-1-propyl group, 1-phenyl-2-propyl group, 2-phenyl-2-propyl group, 1-phenyl-3-propyl group, 1-phenyl-4-butyl group, 1-phenyl-5-pentyl group and 1-phenyl-6-hexyl group. The hydrocarbon groups are preferably hydrocarbon groups having 1 to 20 carbon atoms, more preferably hydrocarbon groups having 1 to 12 carbon atoms, still more preferably hydrocarbon groups having 1 to 8 carbon atoms.

Q¹ in -OSO₂Q¹ is a hydrocarbon group which may have a substituent; the hydrocarbon group includes the above-mentioned hydrocarbon groups; and the substituent includes, for example, a fluorine atom and a nitro group.
Groups denoted by -OSO₂Q¹ are exemplified by an alkylsulfonate group, arylsulfonate group and arylalkylsulfonate group; the alkylsulfonate group is exemplified by a methanesulfonate group, ethanesulfonate group and trifluoromethanesulfonate group; the arylsulfonate group is exemplified by a benzenesulfonate group, p-toluenesulfonate group, p-nitrobenzenesulfonate group and o-nitrobenzenesulfonate group; and the arylalkylsulfonate group is exemplified by a benzylsulfonate group. The groups denoted by -OSO₂Q¹ preferably include a trifluoromethanesulfonate group, benzenesulfonate group, p-toluenesulfonate group and p-nitrobenzenesulfonate group, and more preferably a trifluoromethanesulfonate group.

Q² in -B(OQ²)₂ is a hydrogen atom or a hydrocarbon group which may have a substituent; two Q²s may be the same or different, and may bond with each other to form a ring. The hydrocarbon group includes the above-mentioned hydrocarbon groups, preferably an alkyl group, more preferably a methyl group, ethyl group, propyl group, isopropyl group, butyl group, isobutyl group, pentyl group, hexyl group and nonyl group, still more preferably a methyl group, ethyl group, propyl group, butyl group, pentyl group and hexyl group. In the case of forming a ring, bifunctional hydrocarbon groups composed of two Q²s are preferably a 1,2-ethylene group, 1,1,2,2-tetramethyl-1,2-ethylene group, 1,3-propylene group, 2,2-dimethyl-1,3-propylene group and 1,2-phenylene group. The substituent includes, for example, an amino group.
Groups denoted by -B(OQ²)₂ are exemplified by groups represented by the following chemical formulae.

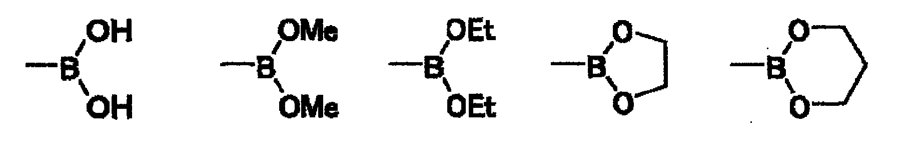

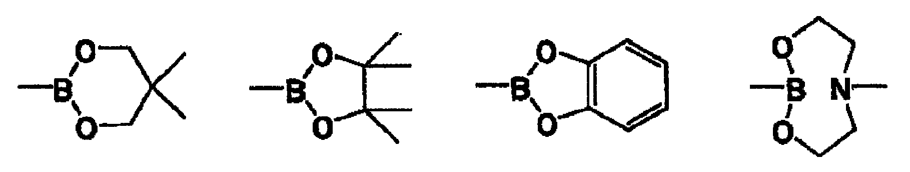

Q³ in -Si(OQ³)₃ is a hydrocarbon group which may have a substituent; and three Q³s may be the same or different. The hydrocarbon groups include the above-mentioned hydrocarbon groups, preferably an alkyl group, more preferably a methyl group, ethyl group, propyl group, isopropyl group, butyl group, isobutyl group, pentyl group, hexyl group and nonyl group, still more preferably a methyl group, ethyl group, propyl group, butyl group, pentyl group and hexyl group. The substituent includes, for example, an amino group and alkoxy group.
Groups denoted by -Si(OQ³)₃ include, for example, a trimethoxysilyl group, tri(n-butyloxy)silyl group and tri(methoxymethyloxy)silyl group.

Q⁴ in -Sn(OQ⁴)₃ is a hydrocarbon group which may have a substituent; and three Q⁴s may be the same or different. The hydrocarbon groups include the above-mentioned hydrocarbon groups, preferably an alkyl group, more preferably a methyl group, ethyl group, propyl group, isopropyl group, butyl group, isobutyl group, pentyl group, hexyl group and nonyl group, still more preferably a methyl group, ethyl group, propyl group, butyl group, pentyl group and hexyl group. The substituent includes, for example, an amino group and alkoxy group.
Groups denoted by -Sn(Q⁴)₃ include, for example, a tri(n-butyl)tin group and triphenyltin group.

Z¹ in Z¹(Z²)m is a metal atom or a metal ion; Z² is a counter ion thereof; and m is an integer of not less than 0. Specific examples of Z¹ include atoms or ions such as Li, Na, K, Rb, Cs, Be, Mg, Ca, Sr, Ba, Al, Ga, In, Tl, Pb, Sc, Ti, V, Cr, Mn, Fe, Co, Ni, Cu, Zn, Y, Zr, Nb, Mo, Tc, Ru, Rh, Ag, Cd, La, Ce, Sm, Eu, Hf, Ta, W, Re, Os, Ir, Pt, Au and Hg. They preferably include Li, Na, K, Rb, Cs, Be, Mg, Ca, Sr, Ba, Al, Ga, In, Tl, Pb, Sc, Ti, Cu, Zn, Y, Zr, Ag and Hg, more preferably Li, Na, K, Rb, Cs, Be, Mg, Ca, In, Tl, Pb, Cu, Zn, Zr, Ag and Hg, still more preferably Li, Na, K, Mg, Ca, Cu and Zn.

As Z², a conjugate base of a Broensted acid is usually used. Specific examples include a fluoride ion, chloride ion, bromide ion, iodide ion, sulfate ion, nitrate ion, carbonate ion, perchlorate ion, tetrafluoroborate ion, hexafluorophosphate ion, methanesulfonate ion, trifluoromethanesulfonate ion, toluenesulfonate ion, acetate ion, trifluoroacetate ion, propionate ion, benzoate ion, hydroxide ion, oxide ion, methoxide ion and ethoxide ion. The specific examples are preferably a chloride ion, bromide ion, iodide ion, sulfate ion, nitrate ion, carbonate ion, methanesulfonate ion, trifluoromethanesulfonate ion, toluenesulfonate ion, acetate ion, trifluoroacetate ion, propionate ion and benzoate ion, more preferably a chloride ion, bromide ion, iodide ion, methanesulfonate ion, trifluoromethanesulfonate ion, toluenesulfonate ion, acetate ion, trifluoroacetate ion, propionate ion and benzoate ion, still more preferably a chloride ion, bromide ion, iodide ion, methanesulfonate ion, trifluoromethanesulfonate ion, acetate ion and trifluoroacetate ion.

m is determined so that an aromatic compound represented by the above-mentioned general formula (1) has the electric neutrality. In the case of X1 or X2 being Z¹(Z²)m, that is, in the case where an aromatic compound represented by the general formula (1) is represented by the general formula (1-1) or (1-2) below: 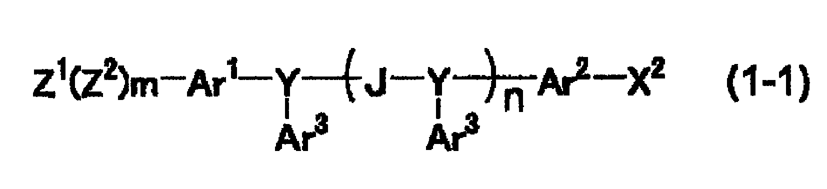 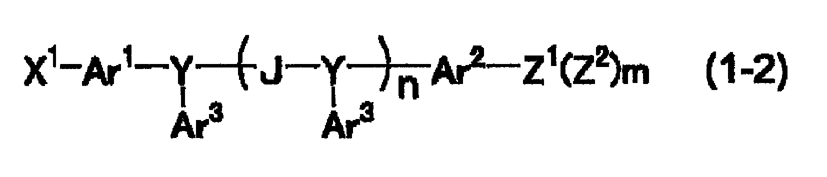 , regarding the Z¹(Z²)m moiety as having a valence number of +1, and the moiety represented by the general formula (1-1b) or (1-2b) below: 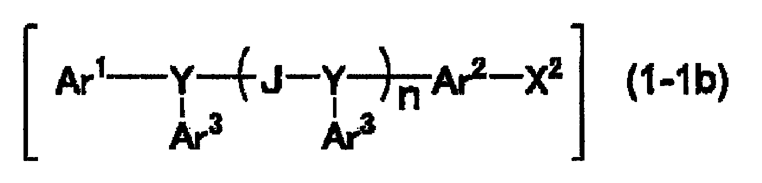 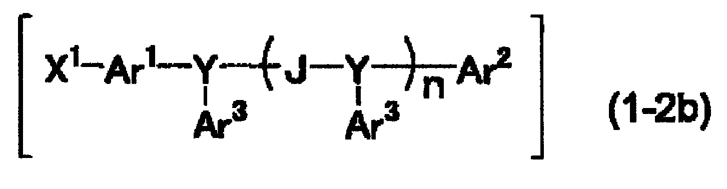 as having a valence number of -1, the Z¹(Z²)m moiety and the remainder moiety are more preferably regarded as being ionically bonded.
Atomic groups denoted by Z¹(Z²) m are exemplified by a zinc halide group, an alkaline metal atom and an alkaline earth metal halide group. Zinc halide groups are exemplified by a zinc chloride group, zinc bromide group and zinc iodide group, and preferably include a zinc chloride group and zinc bromide group. Alkaline metal atoms are exemplified by lithium, sodium and potassium, and preferably include lithium and sodium. Alkaline earth metal halide groups are exemplified by a magnesium chloride group, magnesium bromide group, magnesium iodide group, calcium chloride group, calcium bromide group and calcium iodide group, and preferably include a magnesium chloride group, magnesium bromide group and magnesium iodide group.

Metal acetylide groups are exemplified by a copper acetylide group, zinc acetylide group, magnesium chloride acetylide group, magnesium bromide acetylide group, magnesium iodide acetylide group and tri-n-butyltin acetylide group.

Characteristic groups denoted by X¹ in the above-mentioned general formulae preferably include a chlorine atom, bromine atom, iodine atom, -OSO₂Q¹, -B(OQ²)₂, -Si(OQ³)₃, -Sn(Q⁴)₃ and Z¹(Z²)m; more preferably a halogen atom and -OSO₂Q¹, still more preferably a chlorine atom, bromine atom, iodine atom, trifluoromethanesulfonate group, benzenesulfonate group, p-toluenesulfonate group and p-nitrobenzenesulfonate group; even still more preferably a chlorine atom, bromine atom, iodine atom and trifluoromethanesulfonate group; even still more preferably a chlorine atom, bromine atom and trifluoromethanesulfonate group.

Characteristic groups denoted by X² in the above-mentioned general formulae preferably include a chlorine atom, bromine atom, iodine atom, -OSO₂Q¹, -B(OQ²)₂, -Si(OQ³)₃, -Sn(Q⁴)₃ and Z¹ (Z²)m, metal acetylide group and terminal acetylene group; more preferably a chlorine atom, bromine atom, iodine atom, -OSO₂Q¹, -B(OQ²)₂, trimethoxysilyl group, tri(n-butyloxy)silyl group, tri(n-butyl)tin group, triphenyltin group and Z¹(Z²)m; still more preferably a chlorine atom, bromine atom, iodine atom, trifluoromethanesulfonate group, benzenesulfonate group, p-toluenesulfonate group, p-nitrobenzenesulfonate group, -B(OQ²)₂, trimethoxysilyl group, tri(n-butyloxy)silyl group, tri(n-butyl)tin group, triphenyltin group and Z¹(Z²)m; even still more preferably a chlorine atom, bromine atom, iodine atom, trifluoromethanesulfonate group, benzenesulfonate group, p-toluenesulfonate group, p-nitrobenzenesulfonate group, -B(OQ²)₂, trimethoxysilyl group, tri(n-butyloxy)silyl group, tri(n-butyl)tin group, triphenyltin group, zinc halide group, alkali metal atom and alkaline earth metal halide group; even still more preferably above all, a chlorine atom, bromine atom, iodine atom, trifluoromethanesulfonate group, benzenesulfonate group, p-toluenesulfonate group, p-nitrobenzenesulfonate group, -B(OQ²)₂, trimethoxysilyl group, tri(n-butyloxy)silyl group, tri(n-butyl)tin group, triphenyltin group, zinc halide group and alkaline earth metal halide group.

Combinations of the above-mentioned characteristic groups denoted by X¹ and X² preferably include a chlorine atom and a bromine atom, chlorine atom and iodine atom, chlorine atom and -OSO₂Q¹, chlorine atom and -B(OQ²)₂, chlorine atom and -Si(OQ³)₃, chlorine atom and -Sn(Q⁴)₃, chlorine atom and Z¹(Z²)m, chlorine atom and metal acetylide group, chlorine atom and terminal acetylene group, bromine atom and iodine atom, bromine atom and -OSO₂Q¹, bromine atom and -B(OQ²)₂, bromine atom and -Si(OQ³)₃, bromine atom and -Sn(Q⁴)₃, bromine atom and Z¹(Z²)m, bromine atom and metal acetylide group, bromine atom and terminal acetylene group, iodine atom and -OSO₂Q¹, iodine atom and -B (OQ²)₂, iodine atom and -Si (OQ³)₃, iodine atom and -Sn(Q⁴)₃, iodine atom and Z¹(Z²)m, iodine atom and metal acetylide group, iodine atom and terminal acetylene group, -OSO₂Q¹ and -B(OQ²)₂, -OSO₂Q¹ and -Si(OQ³)₃, -OSO₂Q¹ and -Sn(Q⁴)₃, -OSO₂Q¹ and Z¹(Z²)m, -OSO₂Q¹ and metal acetylide group, and -OSO₂Q¹ and terminal acetylene group; more preferably a chlorine atom and a bromine atom, chlorine atom and iodine atom, chlorine atom and -OSO₂Q¹, chlorine atom and -B(OQ²)₂, chlorine atom and -Si(OQ³)₃, chlorine atom and -Sn(Q⁴)₃, chlorine atom and Z¹(Z²)m, bromine atom and iodine atom, bromine atom and -OSO₂Q¹, bromine atom and -B(OQ²)₂, bromine atom and -Si(OQ³)₃, bromine atom and -Sn(Q⁴)₃, bromine atom and Z¹(Z²)m, iodine atom and -OSO₂Q¹, iodine atom and -B(OQ²)₂, iodine atom and -Si(OQ₃)₃, iodine atom and -Sn(Q⁴)₃, iodine atom and Z¹(Z²)m, -OSO₂Q¹ and -B(OQ²)₂, -OSO₂Q¹ and -Si(OQ³)₃, -OSO₂Q¹ and -Sn(Q⁴)₃, and -OSO₂Q¹ and Z¹(Z²)m; still more preferably a chlorine atom and a bromine atom, chlorine atom and iodine atom, chlorine atom and -OSO₂Q¹, chlorine atom and -B(OQ²)₂, chlorine atom and Z¹(Z²)m, bromine atom and iodine atom, bromine atom and -OSO₂Q¹, bromine atom and -B(OQ²)₂, bromine atom and Z¹(Z²)m, iodine atom and -OSO₂Q¹, iodine atom and -B(OQ²)₂, iodine atom and Z¹(Z²) m, -OSO₂Q¹ and -B (OQ²)₂, -OSO₂Q¹ and Z¹(Z²)m; even still more preferably a chlorine atom and a bromine atom, chlorine atom and iodine atom, chlorine atom and -OSO₂Q¹, chlorine atom and -B(OQ²)₂, bromine atom and iodine atom, bromine atom and -OSO₂Q¹, bromine atom and -B(OQ²)₂, iodine atom and -OSO₂Q¹, iodine atom and -B(OQ²)₂, and -OSO₂Q¹ and -B(OQ²)₂; most preferably a chlorine atom and -B(OQ²)₂, chlorine atom and bromine atom, bromine atom and iodine atom, bromine atom and -OSO₂Q¹, bromine atom and -B(OQ²)₂, iodine atom and -B(OQ²)₂, and -OSO₂Q¹ and -B(OQ²)₂.

Combinations of the above-mentioned characteristic groups denoted by X¹ and X² are preferably those in which X¹ and X¹, X² and X², or X¹ and X² react with each other to produce a bond, more preferably those in which X¹ and X² react with each other to form a bond.

Among the combinations in which X¹ and X² react with each other to form a bond, combinations in which X¹ and X¹, and X² and X² do not react with each other not to produce a bond are further preferable.

Arylene groups denoted by Ar¹, Ar² and J are atomic groups produced by removing two hydrogen atoms from an aromatic hydrocarbon, and also include those which have a condensed ring and those in which two or more independent benzene rings or condensed rings are directly bonded. The arylene groups may have a substituent.
The substituents include an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, fluorine atom, acyl group, acyloxy group, imine residue, amide group, acid imide group, monovalent heterocyclic group, carboxyl group, substituted carboxyl group, nitro group and cyano group, and include an alkyl group, alkoxy group, alkylthio group, aryl group, aryloxy group, arylthio group, arylalkyl group, arylalkoxy group, arylalkylthio group, substituted amino group, fluorine atom, nitro group and cyano group, more preferably an alkyl group, aryl group and arylalkyl group.
The substituents in arylene groups may bond with each other substituent to form a ring.
The number of carbon atoms in a moiety except substituents of an arylene group is usually about 6 to 60, preferably 6 to 20. The total number of carbon atoms in an arylene group including substituents is usually about 6 to 100.
The arylene groups are exemplified by a phenylene group (for example, the below chemical formulae 1 to 3), a naphthalenediyl group (the below chemical formulae 4 to 13), an anthracenediyl group (the below chemical formulae 14 to 19), a biphenyldiyl group (the below chemical formulae 20 to 25), a fluorenediyl group (the below chemical formulae 36 to 38), a terphenyldiyl group (the below chemical formulae 26 to 28), and a condensed-ring compound group (the below chemical formulae 29 to 35).

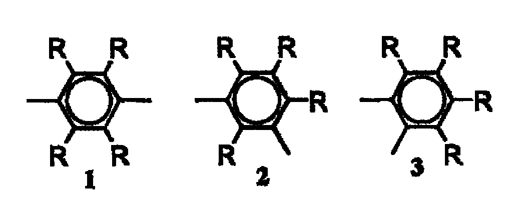

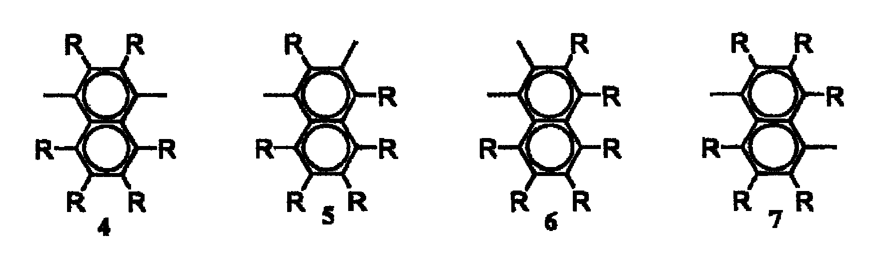

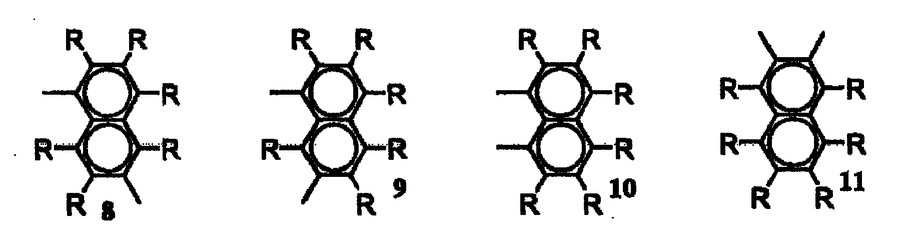

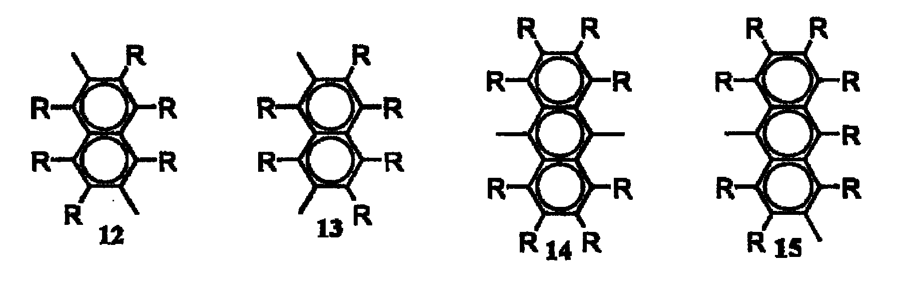

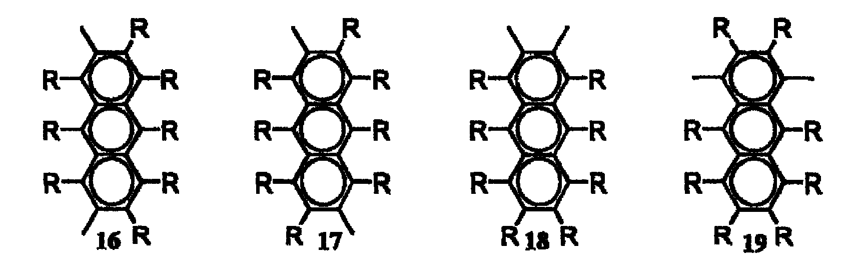

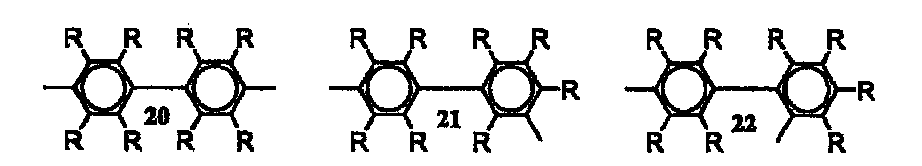

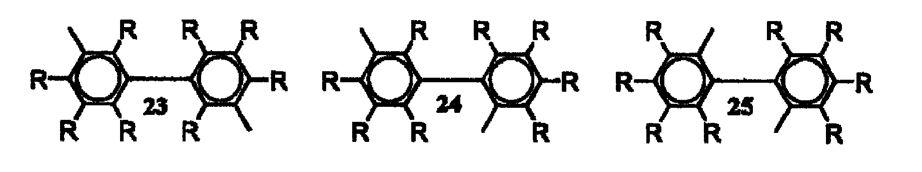

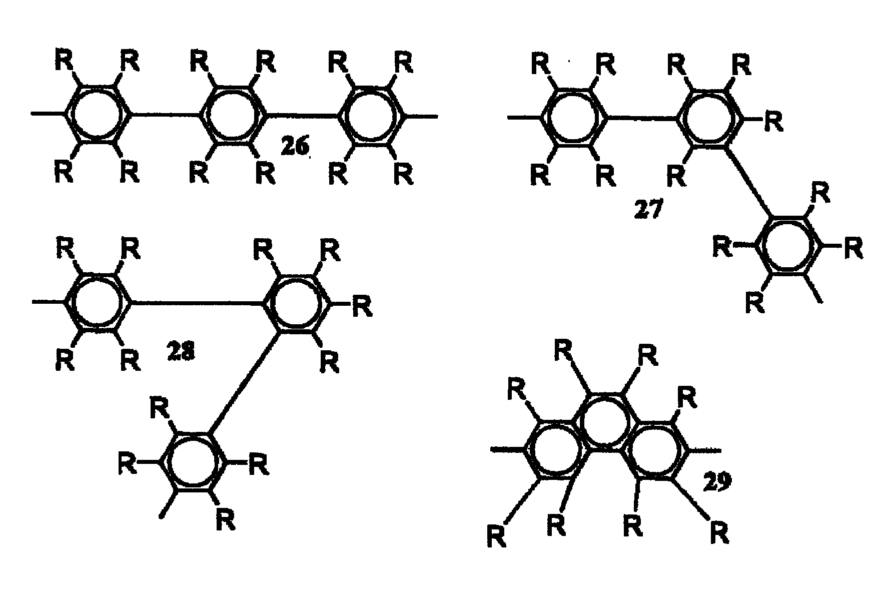

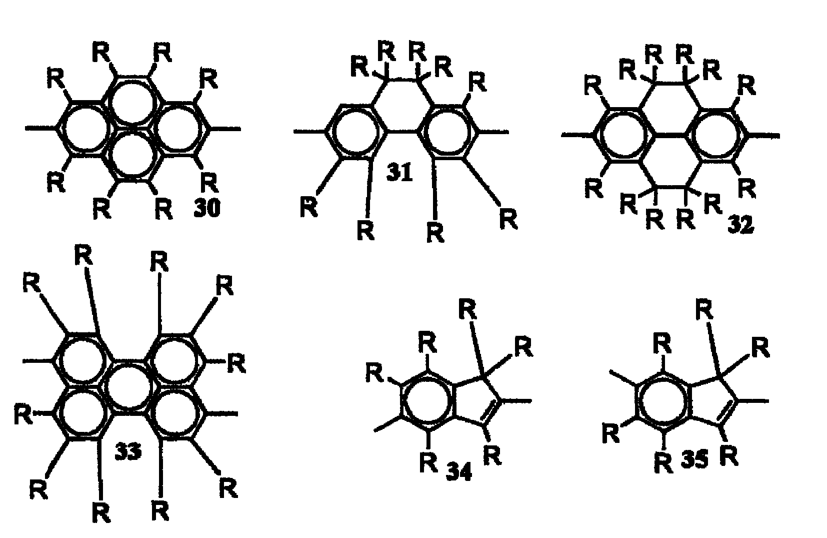

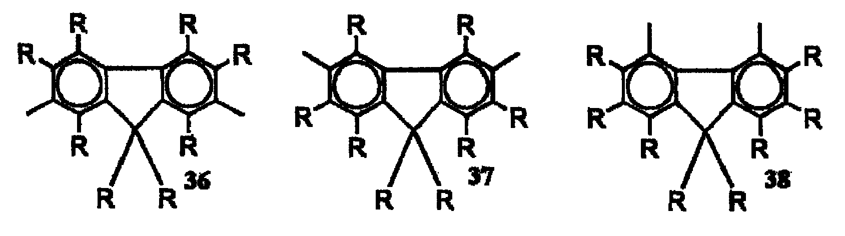

(wherein, R denotes a hydrogen atom or a substituent which the above-mentioned arylene groups may have; and Rs present in plural numbers may be the same or mutually different, and may bond with each other to form a ring.)

Arylene groups denoted by Ar¹ and Ar² are preferably a phenylene group (chemical formulae 1 to 3), a naphthalenediyl group (chemical formulae 4 to 13), a biphenylene group (chemical formulae 20 to 25), a terphenylene group (chemical formulae 26 to 28) and a fluorenediyl group (chemical formulae 36 to 38), more preferably a phenylene group (chemical formulae 1 to 3), a naphthalenediyl group (chemical formulae 4 to 13) and a biphenylene group (chemical formulae 20 to 25), still more preferably a phenylene group (chemical formulae 1 to 3) and most preferably a 1,4-phenylene group (chemical formula 1).

An arylene group denoted by J is preferably a phenylene group (chemical formulae 1 to 3), a naphthalenediyl group (chemical formulae 4-13), a biphenylene group (chemical formulae 20 to 25), a terphenylene group (chemical formulae 26 to 28) and a fluorenediyl group (chemical formulae 36 to 38), more preferably a phenylene group (chemical formulae 1 to 3), a naphthalenediyl group (chemical formulae 4 to 13) and a biphenylene group (chemical formulae 20 to 25), still more preferably a phenylene group (chemical formulae 1 to 3) and a biphenylene group (chemical formulae 20 to 25) and most preferably 1,4-phenylene group (chemical formula 1) and 1,4-biphenylene group (chemical formula 20).

An aryl group denoted by Ar³ is an atomic group produced by removing one hydrogen atom from an aromatic hydrocarbon, and also includes that which has a condensed ring and that in which two or more independent benzene rings or condensed rings are bonded directly or through a vinylene group and the like. The aryl group may have a substituent.
The substituent includes an alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, fluorine atom, acyl group, acyloxy group, imine residue, amide group, acid imide group, monovalent heterocyclic group, carboxyl group, substituted carboxyl group, nitro group and cyano group, preferably an alkyl group, alkoxy group, alkylthio group, aryl group, aryloxy group, arylthio group, arylalkyl group, arylalkoxy group, arylalkylthio group, substituted amino group, fluorine atom, nitro group and cyano group, more preferably an alkyl group, aryl group and arylalkyl group.
The aryl groups are exemplified by those produced by bonding arylene groups denoted by the above-mentioned Ar¹, Ar² and J with one hydrogen atom. The number of carbon atoms in a moiety except substituents of an aryl group is usually about 6 to 60, preferably 6 to 20. An aryl group denoted by Ar³ is preferably a phenyl group, naphthyl group and fluorenyl group, more preferably a phenyl group and naphthyl group, most preferably a phenyl group.

Ar¹, Ar² and Ar³ may be bonded with each other through a substituent or directly.

A trivalent atomic group denoted by Y, in which a hydrogen atom or a hydrocarbon group is optionally added to a nitrogen atom, carbon atom, boron atom or silicon atom, is exemplified by N, CH, CQ⁵, B, SiH and SiQ⁶ (wherein, Q⁵ denotes a hydrocarbon group which may have a substituent; and Q⁶ denotes a hydrocarbon group which may have a substituent). The trivalent atomic group preferably includes N, B, CH and SiH, more preferably N, B and CH, still more preferably N.

Q⁵ is a hydrocarbon group which may have a substituent and the hydrocarbon group includes the above-mentioned hydrocarbon groups. The substituent includes an alkoxyl group, alkylthio group, aryloxy group, arylthio group, arylalkoxy group, arylalkylthio group, amino group, substituted amino group, silyl group, substituted silyl group, fluorine atom, acyl group, acyloxy group, imine residue, amide group, acid imide group, monovalent heterocyclic group, carboxyl group, nitro group and cyano group.

Q⁶ is a hydrocarbon group which may have a substituent and the hydrocarbon group includes the above-mentioned hydrocarbon groups. The substituent includes an alkoxyl group, alkylthio group, aryloxy group, arylthio group, arylalkoxy group, arylalkylthio group, amino group, substituted amino group, silyl group, substituted silyl group, fluorine atom, acyl group, acyloxy group, imine residue, amide group, acid imide group, monovalent heterocyclic group, carboxyl group, nitro group and cyano group.

In the general formulae (1), (1-1), (1-2), (1-1b) and (1-2b), n denotes an integer of not less than 0, and its upper limit is not especially limited, including the case where the compound according to the present invention is a polymer or an oligomer. The upper limit of the integer denoted by n, since it is preferable that the compound according to the present invention dissolves in an organic solvent in consideration of using the compound in various reactions, n is preferably an integer of not more than 10,000, more preferably an integer of not more than 1,000, still more preferably an integer of not more than 100, especially preferably an integer of not more than 10. n is most preferably 0 or 1 in view of easiness of the synthesis.

Compounds represented by the general formula (1) are preferably exemplified by compounds represented by the following chemical formulae (2), (3) and (4): 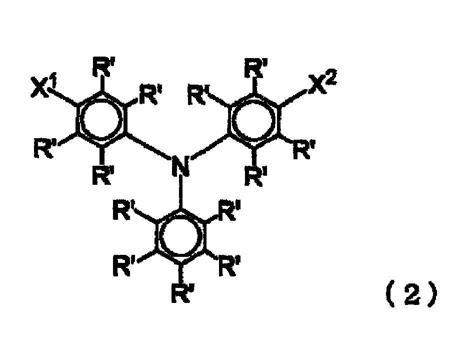 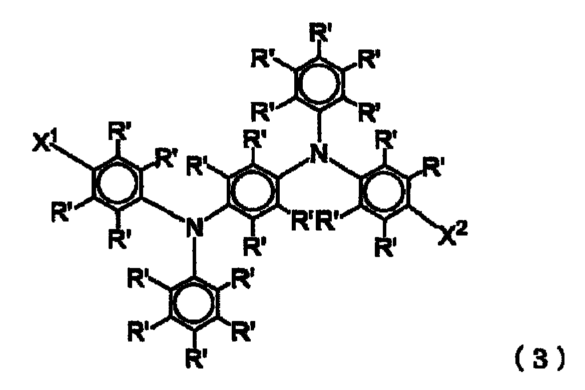 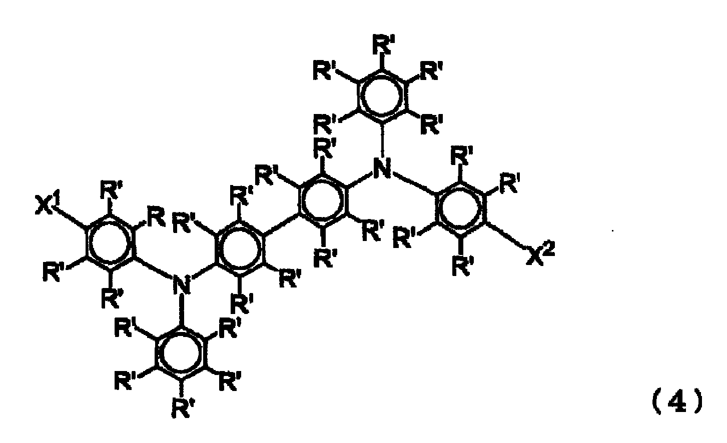 (wherein, R' denotes a hydrogen atom or an alkyl group having 1 to 20 carbon atoms; and R's present in plural numbers may be the same or different from one another).

When the compound according to the present invention represented by the above-mentioned general formula (1) is used for various reactions, the compound according to the present invention is preferably used in a state of being diluted with an organic solvent for various reactions. The organic solvents are not especially limited as long as the solvent can dissolve not less than 0.1 wt.% of the compound according to the present invention, and are preferably exemplified by chlorine-based solvents such as chloroform, methylene chloride, 1,2-dichloroethane, 1,1,2-trichloroethane, chlorobenzene and o-dichlorobenzene; ether solvents such as tetrahydrofuran, dioxane and anisole; aromatic hydrocarbon solvents such as toluene and xylene: aliphatic hydrocarbon solvents such as cyclohexane, methylcyclohexane, n-pentane, n-hexane, n-heptane, n-octane, n-nonane, and n-decane; ketone solvents such as acetone, methyl ethyl ketone, cyclohexanone, benzophenone and acetophenone; ester solvents such as ethyl acetate, butyl acetate, ethyl cellosolve acetate, methyl benzoate and phenyl acetate; polyhydric alcohols and their derivatives such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerol and 1,2-hexanediol; alcohol solvents such as methanol, ethanol, propanol, isopropanol and cyclohexanol; sulfoxide solvents such as dimethyl sulfoxide; and amide solvents such as N-methyl-2-pyrrolidone and N,N-dimethylformamide. Among them, chloroform, methylene chloride, 1,2-dichloroethane, tetrahydrofuran, dioxane, anisole, toluene, xylene, cyclohexane, n-hexane, acetone, methyl ethyl ketone, ethyl acetate, ethylene glycol, ethylene glycol monoethyl ether, ethylene glycol monomethyl ether, dimethoxyethane, propylene glycol, diethoxymethane, methanol, ethanol, propanol, isopropanol, cyclohexanol, dimethyl sulfoxide, N-methyl-2-pyrrolidone and N,N-dimethylformamide are more preferable. Chloroform, methylene chloride, 1,2-dichloroethane, tetrahydrofuran, dioxane, toluene, dimethoxyethane, dimethyl sulfoxide and N,N-dimethylformamide are further preferable. These organic solvents may be used singly or in a combination of two or more.

The purity of the compound according to the present invention represented by the above-mentioned general formula (1) depends on purities required for each type of reaction using as a raw material the compound according to the present invention. When the compound according to the present invention is used for each type of reaction, each content of compounds represented by the following general formulae (2) and (3) : 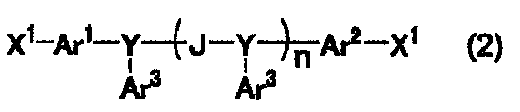 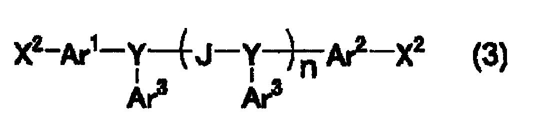 (wherein, X¹, X², Ar¹, Ar², Ar³, Y, J and n denote the same meanings as the above-mentioned), in each of which formulae characteristic groups involved in the reaction denoted by X¹ or X² are the same, which compounds will decrease the purity of each compound obtained as a result of the reaction, is preferably not more than 40 mol% to the compound according to the present invention, more preferably not more than 20 mol%, still more preferably not more than 10 mol%, even still more preferably not more than 5 mol%, and even still more preferably, above all, not more than 1 mol%. When the purity required for each reaction using the compound according to the present invention as a raw material is higher than the above, of course, the compound preferably satisfies the requirement.

The compound according to the present invention represented by the above-mentioned general formula (1) can be synthesized; for example, by synthesizing a compound represented by the following general formula (4): 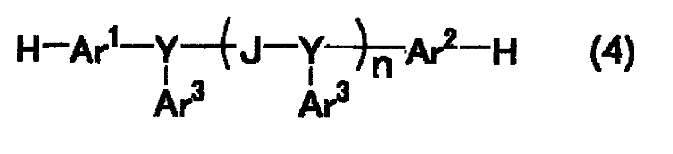 by a well-known method and then synthesizing the target compound by the following methods, (Method A) to (Method F), or by a combination method thereof.

(Method A) The compound represented by the general formula (4) is monohalogenated with bromine, N-bromosuccinimide, N-iodosuccinimide, iodine monochloride or the like and optionally using an acid catalyst; then another halogen atom is introduced by the similar reaction, whereby a compound whose combination of characteristic groups becomes halogen-halogen is synthesized.

(Method B) The compound represented by the general formula (4) is dihalogenated with bromine, N-bromosuccinimide, N-iodosuccinimide, iodine monochloride or the like and optionally using an acid catalyst, and then is mono-metalated by an alkaline metal by using n-BuLi, metallic sodium or the like, whereby a compound whose combination of characteristic groups becomes halogen-alkaline metal is synthesized.

(Method C) The compound represented by the general formula (4) is dihalogenated with bromine, N-bromosuccinimide, N-iodosuccinimide, iodine monochloride or the like and optionally using an acid catalyst, and then is mono-metalated by a halogenated alkaline earth metal salt by using Mg, MgBr₂, ZnCl₂ or the like, whereby a compound whose combination of characteristic groups becomes halogen-halogenated alkaline earth metal is synthesized.

(Method D) Water, hydrochloric acid or the like is acted on the compound whose combination of characteristic groups has become halogen-alkaline metal or halogen-halogenated alkaline earth metal to obtain a monohalogenated compound; then another halogen atom is introduced to the monohalogenated compound with bromine, N-bromosuccinimide, N-iodosuccinimide, iodine monochloride or the like and optionally using an acid catalyst, whereby a compound whose combination of characteristic groups becomes halogen-halogen is synthesized.

(Method E) Trimethyl borate or the like is acted on the compound whose combination of characteristic groups has become halogen-alkaline metal or halogen-halogenated alkaline earth metal; then the resultant is hydrolyzed to form a boric acid salt, whereby a compound whose combination of characteristic groups becomes halogen-boric acid is synthesized.

(Method F) 2-isopropoxy-4,4,5,5-tetramethyl-1,3,2-dioxabororan or the like is acted on the compound whose combination of characteristic groups has become halogen-alkaline metal or halogen-halogenated alkaline earth metal to esterify to form a boric ester, whereby a compound whose combination of characteristic groups becomes halogen-boric ester is synthesized.

The compound according to the present invention represented by the general formula (1), by utilizing a difference between characteristic groups denoted by X¹ and X², can be derivatized into compounds, polymers whose structures are highly controlled.

For example, as a combination of X¹ and X² in the general formula (1), by employing a combination of a halogen atom, selected from a chlorine atom, bromine atom and iodine atom, and -B(OQ²)₂, halogen atom and -Si(OQ³)₃, halogen atom and -Sn(Q⁴)₃, halogen atom and Z¹(Z²)m, halogen atom and metal acetylide group, halogen atom and terminal acetylene group, -OSO₂Q¹ and -B(OQ²)₂, -OSO₂Q¹ and -Si(OQ³)₃, -OSO₂Q¹ and -Sn(Q⁴)₃, -OSO₂Q¹ and Z¹(Z²)m, -OSO₂Q¹ and metal acetylide group, or -OSO₂Q¹ and terminal acetylene group, a polymer in which X¹ and X² are reacted with and bonded with each other can be produced by a well-known method.

By employing a combination of a halogen atom and a halogen atom, halogen atom and -OSO₂Q¹, halogen atom and -B(OQ²)₂, halogen atom and -Si(OQ³)₃, halogen atom and -Sn(Q⁴)₃, halogen atom and Z¹(Z²)m, halogen atom and metal acetylide group, halogen atom and terminal acetylene group, -OSO₂Q¹ and -OSO₂Q¹, -OSO₂Q¹ and -B(OQ²)₂, -OSO₂Q¹ and -Si(OQ³)₃, or -OSO₂Q¹ and -Sn(Q⁴)₃, -OSO₂Q¹ and Z¹(Z²)m, -OSO₂Q¹ and metal acetylide group, or -OSO₂Q¹ and terminal acetylene group, and further by reacting with a compound which has a reactivity with only either one of the characteristic groups by suitably selecting the reaction condition, a compound in which X¹ and X² are each converted to a different group can be obtained.

### Examples

Hereinafter, examples will be shown to describe the present invention further in detail, but the scope of the present invention is not limited thereto.

(LC analysis)
Measurement apparatus: Shimadzu LC-10AVp
Measurement conditions: Kaseisorp LC-ODS 2000-3, φ4.6 µm×100 mm;
Solution A: distilled water, Solution B: acetonitrile Gradient
Solution B: 60% → (10 min) → 100% → (10 min) → 100% Sample concentration: 1.0 mg/mL (THF solution) Injection amount: 1 µL
Detection wavelength: 254 nm
Column temperature: 40°C
(NMR measurement)
NMR measurement was carried out by making a solution of a compound in a deuterated chloroform or a deuterated tetrahydrofuran and using an INOVA300 nuclear magnetic resonance spectrometer, made by Varian, Inc. at room temperature.

### Synthesis Example 1

### <Synthesis of 4-t-butyl-2,6-dimethylbromobenzene>

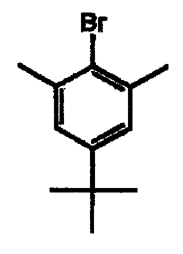

225 g of acetic acid was charged in a 500-ml three-neck flask in an inert atmosphere, and 24.3 g of 5-t-butyl-m-xylene was added thereto. Then, 31.2 g of bromine was added to the solution, and thereafter, the solution was reacted at 15 to 20°C for 3 h.
The reaction solution was added to 500 ml of water, and the deposited precipitate was filtered. The precipitate was rinsed twice with 250 ml of water to obtain 34.2 g of a white solid.
¹H-NMR(300MHz/CDCl₃):
δ(ppm)=1.3[s,9H], 2.4[s,6H], 7.1[s,2H]
MS(FD⁺)M⁺ 241

### <Synthesis of N,N-diphenyl-N-(4-t-butyl-2,6-dimethylphenyl)-amine

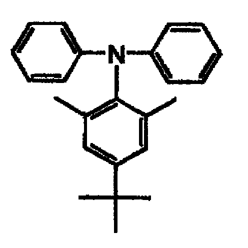

100 ml of a deaerated dehydrated toluene was charged in a 300-ml three-neck flask in an inert atmosphere, and 16.9 g of diphenylamine and 25.3 g of 4-t-butyl-2,6-dimethylbromobenzene were added thereto. Then, 0.92 g of tris(dibenzylideneacetone)dipalladium and 12.0 g of t-butoxysodium were added thereto and thereafter, 1.01 g of tri(t-butyl)phosphine was added thereto. Thereafter, the solution was allowed to react at 100°C for 7 h.
The reaction solution was poured in a saturated brine and extracted with 100 ml of toluene. The toluene layer was rinsed with a dilute hydrochloric acid and a saturated brine, and then the solvent was distilled out to obtain a black solid. The black solid was purified by silica gel column chromatography (hexane/chloroform 9/1) to obtain 30.1 g of a white solid.
¹H-NMR (300MHz/CDCl₃) :δ (ppm)=1.3 [s, 9H], 2.0 [s, 6H], 6.8~7.3 [m, 10H]

### <Synthesis of N,N-bis(4-bromophenyl)-N-(4-t-butyl-2,6-dimethylphenyl)-amine>

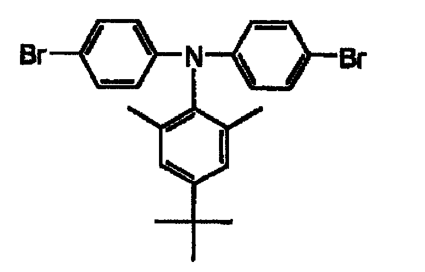

333 ml of a dehydrated N,N-dimethylformamide and 166 ml of hexane were charged in a 1,000-ml three-neck flask in a inert atmosphere, and 29.7 g of the above-mentioned N,N-diphenyl-N-(4-t-butyl-2,6-dimethylphenyl)-amine was dissolved therein. Thereafter, the solution was dropwise added with a solution of 33.6-g N-bromosuccinimide/100-ml N,N-dimethylformamide and allowed to react in an ice bath all day long in the dark.
The reaction solution was concentrated to 200 ml under reduced pressure and added to 1,000 ml of water, and the deposited precipitate was filtered. Further, the obtained crystals were recrystallized twice from DMF/ethanol to obtain 23.4 g of a white solid.
¹H-NMR(300MHz/CDCl₃) :
δ (ppm) = 1.3[s, 9H], 2.0[s,6H], 6.8[d,2H], 7.1[s,2H], 7.3[d,2H],
MS(APCI(+)):M⁺ 488

### (Synthesis of Compound D)

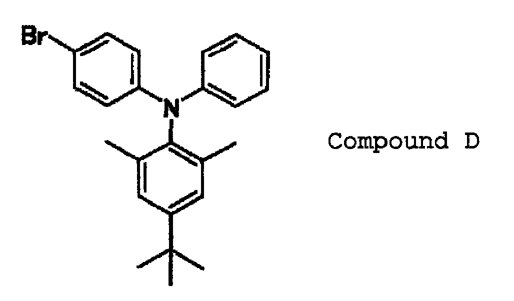

Compound C (20.0 g, 0.041 mol) was charged in a 1-L flask in an argon atmosphere and the atmosphere inside the flask was replaced by argon gas; then, tetrahydrofuran (dehydrated solvent)(80 ml) and diethylether (dehydrated solvent)(400 ml) were charged thereto, and the mixture was stirred and dissolved; thereafter, into the solution which had been cooled in a dry ice-methanol bath at -78°C, a hexane solution of n-BuLi (1.54 M, 29.3 ml, 1.1 MR) was dropwise charged in 30 min. After the temperature of -78°C was held for 1 h, a hexane solution of n-BuLi (1.54 M, 2.6 ml, 0.1 MR) was dropwise charged in 5 min. Further, after the temperature of -78°C was held for 50 min, a hexane solution of n-BuLi (1.54 M, 1.3 ml, 0.05 MR) was again dropwise charged in 5 min. Further, after the temperature of -78°C was held for 1 h, the temperature was raised to -30°C in 30 min, and the reaction mass was added with distilled water (200 ml), stirred and was subjected to liquid-separation. An obtained oil layer was rinsed with distilled water (200 ml), and then dried and concentrated with magnesium sulfate anhydride to obtain an oily substance (20.3 g). The obtained oily substance was purified by a silica gel column whose developing solvent was a mixed solvent of cyclohexanone/chloroform=40/1 (v/v) to obtain Compound D (6.8 g, yield: 40%, LC area percentage: 99.9%) as an oily substance.
¹H-NMR(300MHz,CDCl₃):δ1.32(s,9H), 2.00(s,6H), 6.81-6.98(m,5H), 7.09(s,2H), 7.16-7.27(m,4H)
LC/MS(APPI(+)):M⁺ 407

### Example 1

### (Synthesis of Compound E)

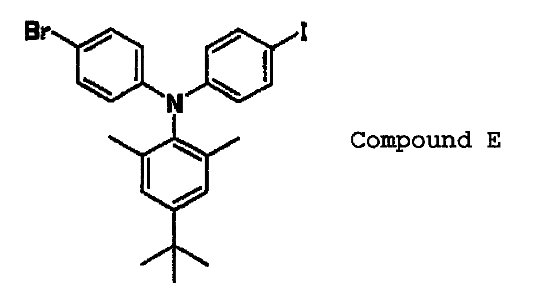

Compound D (14.46 g, 0.0354 mol) and N-iodosuccinimide (9.56 g, 0.0425 mol, 1.2 MR) were charged in a 500-ml flask in an argon atmosphere and argon gas was substituted inside the flask; then, N,N-dimethylformamide (dehydrated solvent)(145 ml) was charged, and the mixture was stirred and dissolved; thereafter, trifluoroacetic acid (2.42 g, 0.0212 mol, 0.6 MR) was charged at room temperature. The temperature of the reaction mass was raised to 50°C, and held for 5 h; then an obtained slurry reaction mass was cooled to room temperature. The reaction mass was added to a mixed solution of distilled water (145 ml) and a saturated sodium sulfite aqueous solution (29 ml) which had separately been prepared. The resultant was extracted with toluene (370 g), rinsed with distilled water (145 ml × twice), and dried and concentrated with magnesium sulfate anhydride to obtain a cream-colored solid (19.9 g). The obtained solid was dispersed in ethyl acetate (60 g), and heated and dissolved at 70°C; methanol (400 g) was dropwise charged in the solution at 70°C, and the mixture was allowed to cool to room temperature; then, the resultant was filtered and dried to obtain Compound E (15.1 g, yield: 80%, LC area percentage: 99.9%) as a white powder.
¹H-NMR(300MHz,CDCl₃):δ1.32(s,9H), 1.99 (s, 6H), 6.72(d,2H), 6.84(d,2H), 7.09(s,2H), 7.28(d,2H), 7.45 (d,2H)
LC/MS(APPI(+)):M⁺ 533.0

### Example 2

### (Synthesis of Compound F)

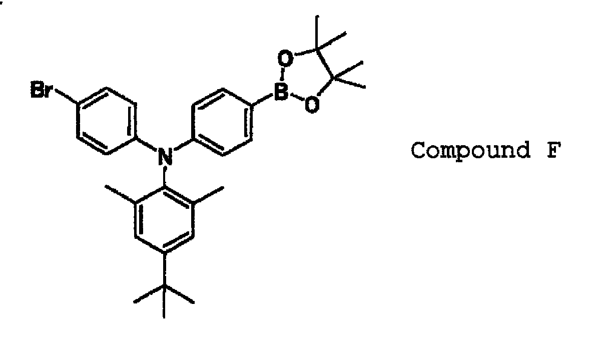

### Synthesis of (4-bromo-phenyl)-(4-tert-butyl-2,6-dimethyl-phenyl)-[4-(4,4,5,5-tetramethyl-[1,3,2]dioxaborolan-2-yl)-phenyl]amine

91.89 g (188.58 mmol) of bis-(4-bromophenyl)-(4-tert-butyl-2,6-dimethyl-phenyl) amine was charged in a dried four-neck flask in an argon atmosphere, and added with 2,750 ml of dehydrated tetrahydrofuran and homogeneously mixed. The reaction solution was cooled to -70°C; 98 ml (150.9 mmol) of a n-hexane solution of 1.54-M n-butyllithium was dropwise charged thereto in 78 min, and the resulting mixture was stirred for 65 min as it was. Then, 35.1 g (188.65 mmol) of 2-isopropoxy-4, 4, 5, 5-tetramethyl-[1,3,2]dioxaborolane was dropwise charged in 60 min at -70°C, and the resulting mixture was stirred for 1 h as it was; thereafter, the solution was raised in temperature to 15 to 20°C, and stirred for 2 h. 1 L of water was charged at room temperature and the resulting mixture was stirred for 1 h, and then tetrahydrofuran was distilled out by reduced-pressure concentration. 2 L of toluene was added to the concentrated suspension and stirred; then, the oil layer and the aqueous layer were separated. The oil layers obtained after the separation three times were combined, and after addition of sodium sulfate anhydride, the oil was stirred. Sodium sulfate anhydride was filtered out; the obtained filtrate was concentrated under reduced pressure to obtain 113.54 g of a white solid. The white solid was purified by silica gel chromatography using toluene and n-hexane as a developing liquid, concentrated, and dried and solidified to obtain 45.5 g of a yellowish white solid. The following operation was seven times repeated: the yellowish white solid was dissolved in 800 ml of tetrahydrofuran; 800 ml of distilled water was dropwise charged at 25°C in 3 h to deposit crystals, and the mixture was filtered after it was stirred for 1 h. The obtained crystals were dried under reduced pressure to obtain the target compound (4-Bromo-phenyl)-(4-tert-butyl-2,6-dimethyl-phenyl)-[4-(4,4,5,5-tetramethyl-[1,3,2]dioxaborolan-2-yl)-phenyl]amine as a white solid (yield amount: 40.2 g, yield rate: 37.8%, LC area percentage: 98.0%). ¹H-NMR:1.32(s,9H), 1.32(s,12H), 1.98(s,6H), 6.87(d,2H), 6.90(d,2H), 7.09(s,2H), 7.28(d,2H), 7.63(d,2H) LC-MS:535.1(M+H)

### INDUSTRIAL APPLICABILITY

The novel aryl compound according to the present invention, which has two characteristic groups different from each other, is useful as raw material for functional materials such as organic EL materials and conductive polymer materials, whose structures are highly controlled, ligands in catalytic reaction and the like.

## Claims

1. A novel aryl compound, **characterized in that** the aryl compound is represented by the following formula (1): 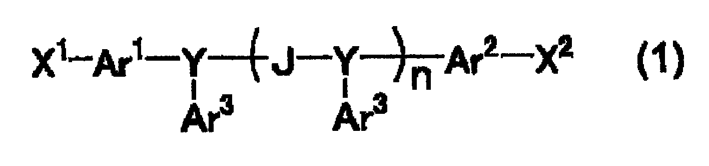 wherein X¹ and X² each independently denote one characteristic group selected from the group consisting of a chlorine atom, a bromine atom, an iodine atom, -OSO₂Q¹, -B(OQ²)₂, -Si(OQ³)₃, -Sn(Q⁴)₃, Z¹(Z²)m, a metal acetylide group and a terminal acetylene group (wherein Q¹ is a hydrocarbon group; Q² is a hydrogen atom or a hydrocarbon group, and two Q²s may be the same or different and may bond with each other to form a ring; Q³ is a hydrocarbon group, and three Q³s may be the same or different; Q⁴ is a hydrocarbon group, and three Q⁴s may be the same or different; Z¹ is a metal atom or a metal ion; Z² is a counter anion thereof; and m denotes an integer of not less than 0); Ar¹ and Ar² each independently denotes an arylene group, and Ar³ denotes an aryl group; Ar¹, Ar² and Ar³ may be bonded with one another directly or through a substituent; Y denotes a trivalent atom group in which a hydrogen atom or a hydrocarbon group is optionally added to a nitrogen atom, a carbon atom, a boron atom or a silicon atom; J denotes an arylene group; n denotes an integer of not less than 0; and in the case where J, Y and Ar³ are each present in plural numbers, they may be each the same or different from one another, and that the aryl compound has characteristic groups denoted by X¹ and X² different from each other.

2. A composition, **characterized in that** the composition comprises the aryl compound according to claim 1 and at least one organic solvent.

3. The composition according to claim 2, wherein the total content of a compound represented by the following formula (2) or (3): 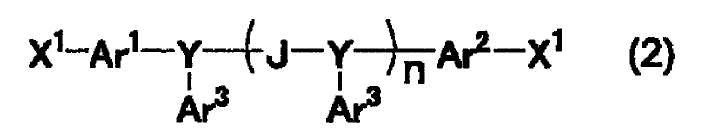 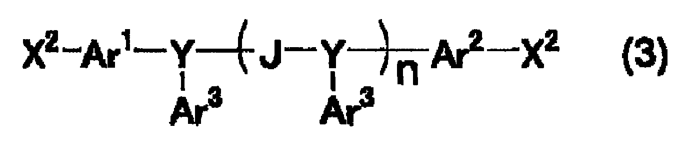 (wherein, X¹, X², Ar¹, Ar², Ar³, Y, J and n have the same meanings as the meanings according to claim 2), which is contained in the composition, is not more than 40 mol% based on the compound represented by the general formula (1).

4. The compound or the composition according to any one of claims 1 to 3, wherein Y denotes a nitrogen atom.

5. The compound or the composition according to any one of claims 1 to 4, wherein n denotes 0 or 1.

6. The compound or the composition according to any one of claims 1 to 5, wherein Ar¹ and Ar² each denotes a phenylene group which may have a substituent; J denotes a phenylene group which may have a substituent or a biphenylene group which may have a substituent; and Ar³ denotes a phenyl group which may have a substituent.

7. The compound or the composition according to any one of claims 1 to 6, wherein the compound or the composition is represented by the following formula (2), (3) or (4) : 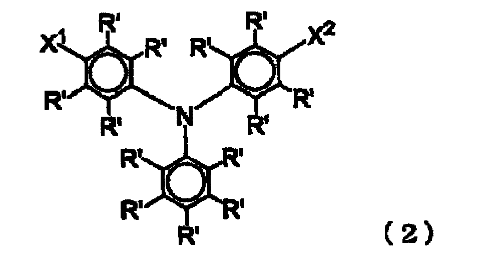 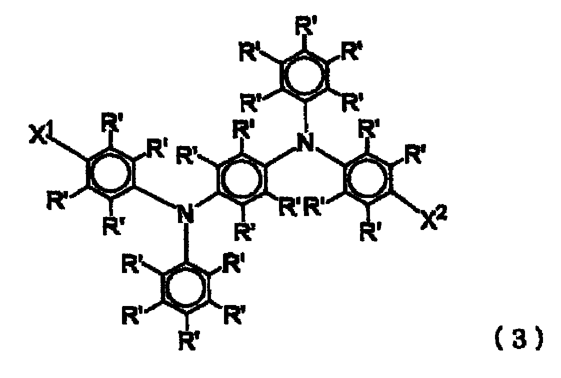 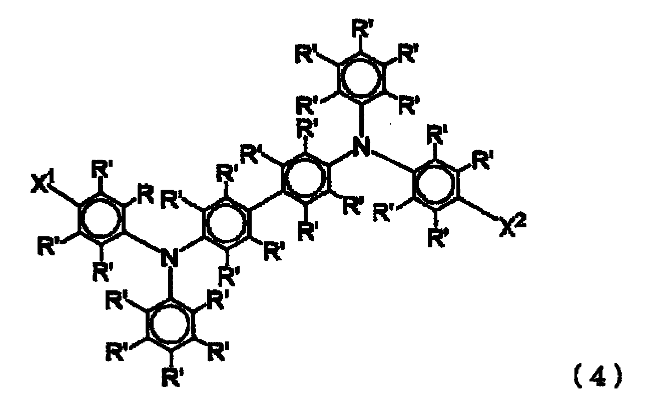 wherein R' denotes a hydrogen atom or an alkyl group having 1 to 20 carbon atoms; and R's present in plural numbers may be the same or different from one another.
